# EUROPEAN PATENT SPECIFICATION

(11) **EP 0 619 020 B1**
(45) Date of publication and mention of the grant of the patent: **08.11.2000**
(21) Application number: 92905134.0
(22) Date of filing: 13.12.1991
(51) Int. Cl.: G01N 33/50, G01N 33/94, G01N 33/53, G01N 30/02, G01N 30/22

(54) **DETERMINATION OF TRICYCLIC ANTIDEPRESSANT DRUGS IN THE PRESENCE OF INTERFERING SUBSTANCES**
BESTIMMUNG TRIZYKLISCHER ANTIDEPRESSIVA IN GEGENWART STÖRENDER SUBSTANZEN
DETECTION DE MEDICAMENTS ANTIDEPRESSEURS TRICYCLIQUES EN PRESENCE DE SUBSTANCES D'INTERFERENCE

(43) Date of publication of application: 12.10.1994
(73) Proprietor: ABBOTT LABORATORIES, Abbott Park, IL 60064-2204 (US)
(72) Inventor: ADAMCZYK, Maciej, Gurnee, IL 60031 (US); FISHPAUGH, Jeffrey, R., Chicago, IL 60657 (US); HARRINGTON, Charles, A., Lake Villa, IL 60046 (US); HARTTER, Daryl, E., Mundelein, IL 60060 (US); HRUSKA, Robert E., Libertyville IL60048 (US)
(74) Representative: Modiano, Guido, Dr.-Ing.
(86) International application number: US9109389
(87) International publication number: WO9312424

(56) References cited:
- EP-A- 0 411 604
- WO-A-90/07115
- US-A- 4 668 620
- US-A- 4 810 635
- US-A- 4 820 416
- US-A- 4 829 012
- Alltech Associates, Inc. Chromatography Catalog No. 150, issued 1988, see pages 58 and 258-263.
- Chemically Abstracts, Volume 106, No. 17, issued 27 April 1987, Y.H. CHO et al., "Determination of Barbiturates in Plasma by Gas Chromatography Flame Photometric Detector after N, N'-Dimethyl-Thiomethyl Derivatization", see page 4, column 2, the Abstract No. 131135C, Arch. Pharmacal. Res. 1986, 9(3), 131-8 (Eng).
- CHEMICAL ABSTRACTS, Volumn 98, No. 7, issued 14 February 1983, K.E. KARLSSON, "Two-Phase Derivatization' of Amitriptyline and Structurally Related Tertiary Amines for Gase Chromatography with Election-Capture Detection", see page 3, column 1, the Abstract No. 46295B, J. Chromatography 1981, 219(3), 373-8 (Eng).
- Biological Abstracts, Volume 68, No. 9, issued 01 November 1979, H.F. PROELSS et al., "High Performance Liquid Chromatographic Simultaneous Determination of Commonly Used Tricyclic Antidepressants", see column 2, the Abstract No. 56243, Clin Chem 24(11): 1948-1953, 1978.
- Aldrich Chemical Company, Inc. Catalog, issued 1986, pages 283, 1194 and 1196, see 28,304-5; 85,731-9; 23,930-5 and 22-341-7.

## Description

### FIELD OF THE INVENTION

The present invention relates to the determination of a tricyclic antidepressant drug which does not contain a sulfur moiety in a test sample also containing one or more analytically interfering substances. In particular, the present invention relates to the pretreatment of a test sample to permit the analytical determination of a tricyclic antidepressant drug which does not contain a sulfur moiety in the presence of one or more interfering substances.

### Background of the Invention

The determination of analytes has become very useful in a variety of fields such as biochemical research, environmental and industrial testing, and especially medical diagnostics. The monitoring of therapeutic drug levels and other analytes in biological fluids such as serum, plasma, whole blood, urine, and the like, has become very useful to provide physicians with information to aid in patient management. The monitoring of such drug levels enables adjustment of patient dosage to achieve optimal therapeutic effects, and helps avoid either subtherapeutic or toxic levels.

A test sample may contain one or more detectable compounds having chemical structures which are substantially similar to, or which resemble, or which could resemble, and therefore be indistinguishable from, the desired analyte of interest. For example, in the area of medical diagnostics where it is important to monitor the level of therapeutic drugs, a number of drugs having substantially similar chemical structures may be administered to a patient wherein a test sample from such patient could contain detectable levels thereof.

In such instances, the analytical determination of the desired analyte may be interfered with by the presence of such one or more additional substances having substantially similar chemical structures to the chemical structure of the analyte of interest to thereby produce inconsistent and inaccurate results. In particular, where the analytical determination involves immunoassay techniques employing antibodies capable of binding to the analyte of interest, such antibodies may also bind or crossreact with such other substances having substantially similar chemical structures. Similarly, where the analytical determination involves high pressure liquid chromatography techniques or thin layer chromatography techniques, the elution profile of the analyte of interest may be indistinguishable from the elution profile of such other substances having substantially similar chemical structures.

U.S. Patent No. 4,810,635 discloses a fluorescence energy transfer immunoassay for the detection of digoxin in a serum sample in which interfering endogenous fluorescence has been eliminated by treatment of the sample with a strong oxidizing agent. U.S. Patent No. 4,668,620 discloses an enzyme-label immunoassay in which a serum sample has been treated with a peracid compound to reduce or eliminate background interference contributed by serum components. U.S. Patent No. 4,829,012 discloses an immunoassay for IgM antibodies in a liquid sample, such as a biological fluid, in which interfering IgG antibodies are modified by the binding of the IgG antibodies to anti-IgG antibodies. Alltech Associates, Inc. Chromatography Catalog #150, issued 1988, pages 58 and 258-263 discloses various chromatography assays for different analyte families involving the pre-treatment with a derivatization agent which allows the differential analysis of a predetermined member(s) of a selected substance family in the presence of other member(s) of the same family. Chemical Abstracts, vol. 106, no. 17, issued 27 April 1987, (Y.H. Cho et al., Arch. Pharmacal. Res. 1986, 9(3), 131-8) discloses the determination of barbiturates such as thiopental in plasma by gas chromatography-flame photometric detector after N,N'-dimethylthiomethyl derivatization. Chemical Abstracts, vol. 98, no. 7, issued 14 February 1983 (K.E. Karlsson, J. Chromatography 1981, 219(3), 373-8) discloses differential analysis of secondary and tertiary amine amitryptiline (a parent compound and its structurally similar metabolites) by gas chromatography following a two-phase derivatization to carbamates. Biological Abstracts, vol. 68, no. 9, issued 01 November 1979 (H.F. Proelss et al., Clin. Chem. 24(11):1948-1953, 1978) discloses a high-performance liquid chromatographic simultaneous determination of five commonly used tricyclic antidepressants (doxepin, desipramine, nortriptyline, imipramine and amitriptyline) in serum in which interference from drugs such as phenothiazines occurs. Aldrich Chemical Company, Inc. Catalog, issued 1986, pages 283, 1194 and 1196 discloses oxidizing agents such as Chloramine-B and Chloramine T which are useful in the present invention.

### Summary of the Invention

The present invention relates to an
immunoassay method for determining the amount of a tricyclic antidepressant drug in a biological test sample, wherein said tricyclic antidepressant drug does not contain a sulfur moiety and wherein said biological test sample includes a sulfur-containing compound having a chemical structure which is similar to said tricyclic antidepressant drug and is immunologically crossreactive with an antibody to said antidepressant drug, said method comprising the steps of:
   (a) forming a pretreatment solution by contacting said biological test sample with an oxidative reagent capable of modifying the chemical structure of said sulfur-containing compound by selectively oxidizing the sulfur moiety of said sulfur-containing compound without modifying the chemical structure of said tricyclic antidepressant drug, whereby crossreactive binding of said antibody to said tricyclic antidepressant drug to said modified sulfur-containing compound is reduced;
   (b) contacting said pretreatment solution with (i) a labeled reagent comprising said tricyclic antidepressant drug or derivative thereof labeled with a fluorescent moiety, and (ii) said antibody capable of binding to said labeled reagent and said tricyclic antidepressant drug from said biological test sample to form a reaction solution therewith, said labeled reagent capable of producing a detectable fluorescence polarization response to the presence of said antibody; and
   (c) detecting the fluorescence polarization response to said reaction solution as a function of the amount of said tricyclic antidepressant drug present in said test sample.

The present invention also relates to a
high pressure liquid chromatography (HPLC) method for determining the amount of a tricyclic antidepressant drug in a biological test sample, wherein said tricyclic antidepressant drug does not contain a sulfur moiety and wherein said biological test sample includes a sulfur-containing compound having a chemical structure similar to the chemical structure of said tricyclic antidepressant drug and an elution profile which interferes with the elution profile of said tricyclic antidepressant drug, said method comprising the steps of:
   (a) forming an admixture of an aliquot of said biological test sample and an internal standard;
   (b) forming a pretreatment solution by contacting said admixture with an oxidative reagent capable of modifying the chemical structure of said sulfur-containing compound by selectively oxidizing the sulfur moiety of said sulfur-containing compound without modifying the chemical structure of said tricyclic antidepressant drug, whereby the elution profile of said modified sulfur-containing compound does not interfere with the elution profile of said tricyclic antidepressant drug;
   (c) contacting said pretreatment solution to an HPLC stationary phase and eluting therefrom with a mobile phase; and
   (d) measuring elution times and absorbances of said internal standard and said tricyclic antidepressant drug as they elute from the stationary phase in order to determine the amount of said tricyclic antidepressant drug in said biological test sample.

Accordingly, by chemically modifying one of such compounds as described by the present invention, methods previously required to permit the selective detection as described above are no longer necessary.

The pretreatment reagent is an oxidative reagent which is useful for the derivatization of sulfur-containing compounds which may be present in a test sample, particularly for the fluorescent polarization immunoassay determination of tricyclic antidepressant drugs in the presence of phenothiazines. As would be understood by one skilled in the art, phenothiazines are often co-prescribed to a patient being treated with a tricyclic antidepressant drug wherein a test sample from such patient would therefore contain levels of both drugs. According to such preferred embodiment, such test sample is contacted with an oxidative reagent, preferably chloramine-T (sodium salt of N-chloro-4-methylbenzene sulfonamide) or chloramine-B (sodium salt of N-chlorobenzene sulfonamide), to selectively modify the chemical structure of the phenothiazines whereby the phenothiazines present in the test sample are selectively oxidized to their corresponding sulfoxides or sulfones. After such pretreatment of the test sample, determination of the tricyclic antidepressant drug can be accomplished
preferably according to fluorescent polarization immunoassay techniques
   wherein crossreactivity of the modified phenothiazine with the antibody to the tricyclic antidepressant drug is substantially reduced.

### Brief Description of the Drawings

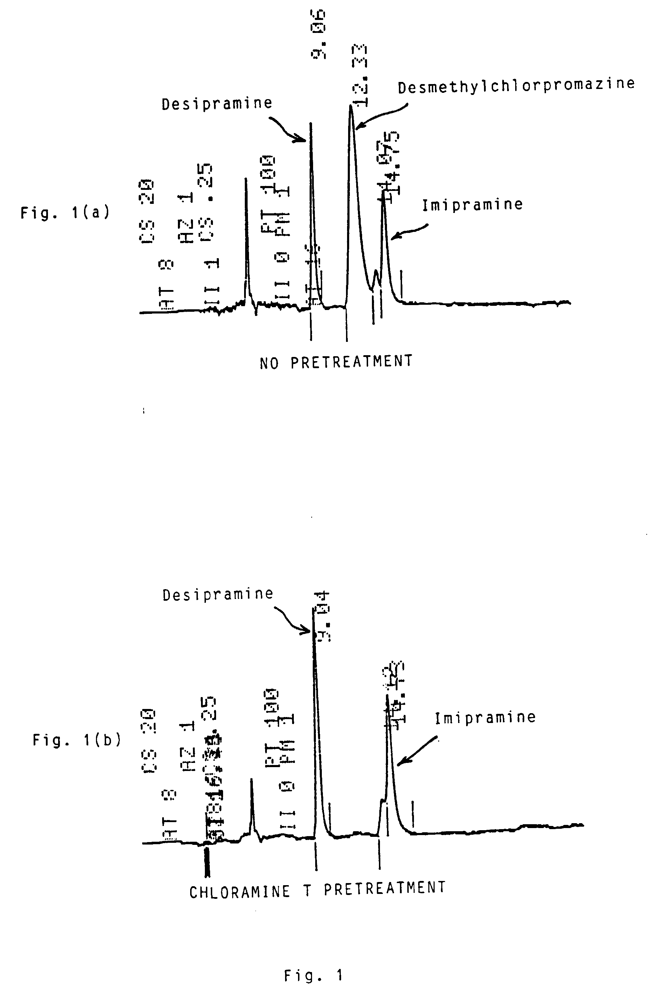

Fig. 1 is a chromatogram which illustrates the elution profiles of tricyclic antidepressant drugs (desipramine and imipramine) and a phenothiazine (desmethylchlorpromazine) prior to [Fig. 1(a)] and subsequent [Fig. 1(b)] to modification of the chemical structure of the phenothiazine according to the present invention.

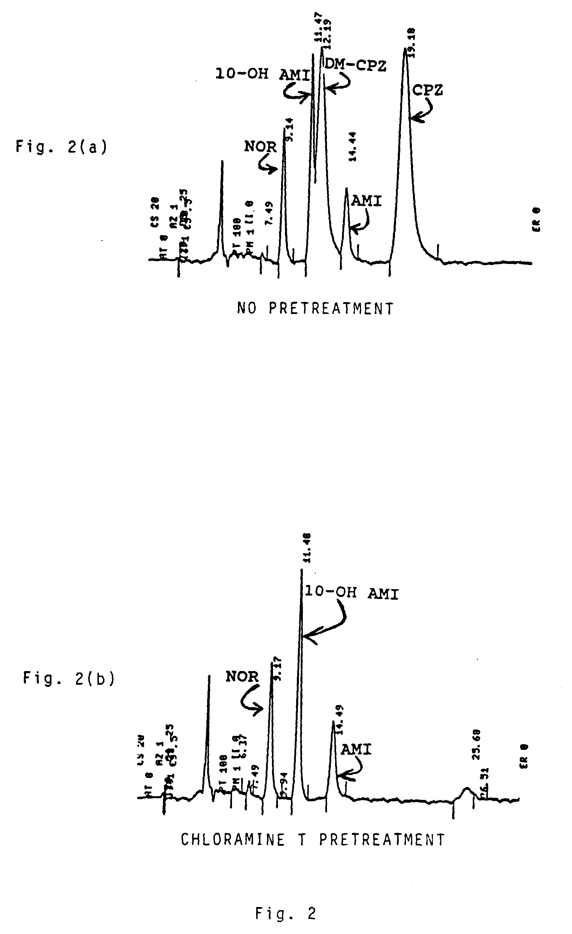

Fig. 2 is a chromatogram which illustrates the elution profiles of tricyclic antidepressant drugs [amitriptyline (AMI), 10-hydroxyamitriptyline (10-OH AMI), and nortriptyline (NOR)] and phenothiazines [desmethylchlorpromazine (DM-CPZ) and chlorpromazine (CPZ)] prior to [Fig. 2(a)] and subsequent to [Fig. 2(b)] modification of the chemical structure of the phenothiazines according to the present invention.

### Detailed Description of the Invention

The present invention is useful for the determination of a tricyclic antidepressant drug which does not contain a sulfur moiety present in a test sample also containing one or more interfering substances which is a sulfur-containing compound having substantially similar chemical structures to, or chemical structures which resemble or could resemble, the analyte of interest. It is to be understood that the analyte of interest and such interfering substances could be substantially dissimilar but, at the same time, could have conformational aspects in which they resemble each other.

In particular, analytes of interest include, but are not intended to be limited to, tricyclic antidepressant drugs such as amitriptyline, nortriptyline, imipramine, desipramine, protriptyline, doxepin, clomipramine and desdoxepin; and benzodiazepines such as diazepam.

In particular, interfering substances include, but are not intended to be limited to phenothiazines such as chloropromazine, desmethylchloropromazine, thioridazine, desmethylthioridazine, sulforidazine and mesoridazine, and thioxanthines such as thiothixene.

According to the present invention, the pretreatment reagent is specific for and is capable of selectively modifying the chemical structure at one or more sites or positions of one or more interfering substances present in a test sample.

It is to be appreciated that once the chemical structure of the interfering substance has been modified according to the present invention, the analytical determination of the analyte can be made. Also according to the present invention, the pretreatment reagent can be selected from a variety of oxidative reagents capable of substantially modifying the chemical structure of the interfering substance. Such reagents include, but are not intended to be limited to N-chlorosulfonamides such as chloramine-T and chloramine-B; hypochlorites such as sodium hypochlorite and hypochlorous acid; hypobromites such as sodium hypobromite and hypobromous acid; peroxides such as sodium hydroperoxide; peracids such as perselenic acid and 3-chloroperbenzoic acid; and oxidative enzymes such as catalase, peroxidase and microperoxidase. For sulfur-containing interfering substances, such as phenothiazines, the pretreatment reagent is preferably selected from the group consisting of sodium hypochlorite, catalase, and perselenic acid, more preferably chloramine-T.

As would be understood by one skilled in the art apprised of the foregoing considerations and as will be described in greater detail in the specific embodiments and examples hereinbelow, selection of a particular pretreatment reagent will depend upon the chemical structure of the analyte of interest and the chemical structure of interfering substances which may be present in a test sample. It is to be understood that in addition to being added or introduced into a test sample from an external source, the oxidative reagent can alternatively be generated or otherwise formed in situ, such as the generation of hydrogen peroxide. For example, in the case of an analytical system containing the analyte of interest, an interfering substance as described herein, selenium dioxide, and glucose oxidase, to which glucose is added, hydrogen peroxide is formed in situ. This is particularly useful for the packaging of reagents in that the packaging and shipment of strong oxidizing agents can be avoided by providing reagents which can form, in situ, the oxidative reagent for use as described herein. In addition, the pretreatment reagent can be presented as a liquid reagent, a dry reagent, or immobilized to a solid support material according to methods known in the art.

Although the pretreatment reagent can be employed in the analytical systems as described above, the pretreatment reagent according to the present invention is especially useful in a fluorescent polarization immunoassay (FPIA) system. For example, in a competitive protein binding or immunoassay, a substance being measured, often referred to as a ligand, competes with a substance of close structural similarity coupled to a detectable moiety, often referred to as a tracer, for a limited number of binding sites on antibodies specific to the portion or portions of the ligand and tracer with structural similarity, shared with an immunogen employed to produce such antibodies. In a fluorescent polarization immunoassay, the detectable moiety component of the tracer is a fluorescent moiety selected from the group consisting of fluoresceins, aminofluoresceins, carboxyfluoresceins, fluoresceinamines, and the like.

According to such FPIA format, the amount of tracer bound to the antibody varies inversely to the amount of ligand present in either the test sample or standard calibrator solution in a predictable manner. The relative, and therefore characteristic, binding affinities of the ligand and the tracer to the antibody binding site, are important parameters of the assay system. In particular, FPIA techniques are based on the principle that a fluorescent tracer, when excited by plane polarized light of a characteristic wavelength, will emit light at another characteristic wavelength (i.e., fluorescence) that retains a degree of the polarization relative to the incident stimulating light that is inversely related to the rate of rotation of the tracer in a given medium. As a consequence of this property, a tracer substance with constrained rotation, such as in a viscous solution phase or when bound to another solution component with a relatively lower rate of rotation, will retain a relatively greater degree of polarization of emitted light than if in free solution. Therefore, within the time frame in which the ligand and tracer compete for binding to the antibody, the tracer and ligand binding rates should yield an appropriate proportion of free and bound tracer with the preservation of important performance parameters such as selectivity, sensitivity, and precision.

For example, for the FPIA determination of imipramine, a test sample is contacted with the pretreatment reagent according to the present invention and with imipramine antiserum in the presence of an appropriately selected fluorescein derivative thereof which is capable of producing a detectable fluorescence polarization response to the presence of imipramine antiserum. Plane polarized light is then passed through the solution to obtain a fluorescent polarization response and the response is detected as a measure of the amount of imipramine present in the test sample when compared to the response of standard calibrators prepared from the native compound.

As described herein, the test sample containing the analyte of interest and such other interfering substances is a biological test sample such as whole blood, serum, plasma, urine, feces, saliva, cerebrospinal fluid and brain tissue.

According to the present invention, there is provided a test kit useful for the fluorescent polarization immunoassay determination of a tricyclic antidepressant drug present in a test sample, wherein said antidepressant drug does not contain a sulfur moiety and wherein said test sample includes a sulfur-containing compound having a chemical structure which is similar to said tricyclic antidepressant drug and is immunologically crossreactive with an antibody to said antidepressant drug, said test kit comprising (a) an oxidative reagent, or reagents which can generate hydrogen peroxide as an oxidative reagent in the test sample, said oxidative reagent being capable of modifying the chemical structure of said sulfur-containing compound by selectively oxidizing the sulfur moiety of said sulfur-containing compound without modifying the chemical structure of said tricyclic antidepressant drug, whereby crossreactive binding of said antibody to said tricyclic antidepressant drug to said modified sulfur-containing compound is reduced and (b) all essential additional reagents for performing the fluorescent polarization immunoassay determination including an antibody to said tricyclic antidepressant drug and a suitable fluorescent tracer compound.

According to the present invention, there is also provided a test kit useful for the chromatographic determination of a tricyclic antidepressant drug present in a test sample, wherein said antidepressant drug does not contain a sulfur moiety and wherein said test sample includes a sulfur-containing compound having a chemical structure which is similar to said tricyclic antidepressant drug and an elution profile which interferes with the elution profile of said tricyclic antidepressant drug, said test kit comprising (a) an oxidative reagent, or reagents which can generate hydrogen peroxide as an oxidative reagent in the test sample, said oxidative reagent being capable of modifying the chemical structure of said sulfur-containing compound by selectively oxidizing the sulfur moiety of said sulfur-containing compound without modifying the chemical structure of said tricyclic antidepressant drug, whereby the elution profile of said modified sulfur-containing compound does not interfere with the elution profile of said tricyclic antidepressant drug; and (b) all essential additional reagents for performing the chromatographic determination including an internal standard. Such test kit may be useful in a high pressure liquid chromatographic determination of said drug or useful in a thin layer chromatographic determination of said drug. The test kit is presented in a commercially packaged form as a combination of one or more containers holding the necessary reagents, as a composition or admixture where the compatibility of the reagents will allow. Particularly preferred is a test kit for the fluorescent polarization immunoassay determination of tricyclic antidepressant drugs, comprising the appropriate pretreatment reagent according to the present invention, an appropriate fluorescent tracer compound, and an appropriate antibody reagent as described above. It is to be understood that the test kit can, of course, include other materials as are known in the art and which may be desirable from a commercial user standpoint, such as buffers, diluents, standards, and the like.

The present invention will now be illustrated, but is not intended to be limited, by the following examples:

### EXAMPLE 1

### Fluorescent Polarization Immunoassay For Imipramine and Desipramine

In a fluorescent polarization immunoassay (FPIA) using an Abbott TDx® analytical system for imipramine (IMI) or desipramine (DMI), phenothiazines such as chlorpromazine may interfere with the determination of IMI and DMI. In particular, antibodies directed to IMI may recognize chlorpromazine or desmethylchlorpromazine as well as imipramine when present in a patient sample. In order to prevent the error in quantitation that would result, the sample is treated with chloramine-T to oxidatively modify the structure of the chlorpromazine or desmethylchlorpromazine to a form much less well recognized by this antitricyclic immune serum.

For the determination of imipramine, the sample (0.100 mL) was rendered basic by the addition of 0.100 mL of 0.25 M NaOH and dissociated from serum proteins by the addition of 0.025 mL of isoamyl alcohol. The mixture was allowed to stand for up to 5.0 minutes, after which time 0.50 mL of decane was added and the sample vortexed vigorously for 1.0 minute. The sample/decane biphasic mixture was then centrifuged for up to 5.0 minutes to clarify those phases. In the case of imipramine, 0.100 mL of the decane upper phase was transferred to a tube containing 0.100 mL of acidic buffer, pH 3, to which 0.010 mL of 20 microgram per mL of chloramine-T had been added. This amount of chloramine-T can completely modify up to 1.0 microgram of chlorpromazine in the original sample. Variations in the quantity of chloramine-T may be made to accommodate differing ranges of phenothiazine concentrations expected to be present in the sample. Table I below illustrates a typical example of results obtained from comparing the analysis of a sample containing, as in this case, 300 ng/mL of chlorpromazine and 128 ng/mL of imipramine, employing an Abbott TDx analytical system. Chlorpromazine crossreacts with the antibody used in the FPIA for imipramine. This quantity of chlorpromazine yielded a significant overestimate of the amount of imipramine actually present in the sample (171%). Treatment of the sample extract with chloramine-T modifies the structure of the chlorpromazine in such a fashion that it is no longer an assay interferant. Inclusion of the chloramine-T treatment removed this overestimate entirely.

**TABLE I**

| Removal Of Chlorpromazine Interferant In FPIA For Imipramine | | |
|---|---|---|
| Sample | Treatment | Mass |
| IMI | NONE | 128 ng/mL |
| IMI+300 ng CPZ | NONE | 219 ng/mL |
| IMI+300 ng CPZ | Chloramine-T | 130 ng/mL |

The procedure described above could also be followed for the analysis of desipramine in the presence of phenothiazines to achieve the elimination of interfering substances as described herein.

### EXAMPLE 2

### High Pressure Liquid Chromatography Determination of Imipramine and Desipramine

In the HPLC analysis of tricyclic antidepressants such as imipramine and desipramine, phenothiazines such as chlorpromazine or its metabolite, desmethylchlorpromazine, may interfere with the analysis of the tricyclic antidepressants or one of their metabolites.

A 1.0 mL sample to which an internal standard substance was added was rendered basic by the addition of 0.200 mL of 1.0 M NaOH followed by the addition of 0.200 mL of isoamyl alcohol to inhibit analyte/protein interaction. Ten mL of heptane was then added and the biphasic mixture shaken for 1.0 hour followed by 30 minutes of centrifugation at about 2000 x g. The heptane layer was then transferred in its entirety to a test tube containing 1.0 mL of an acidic buffer, pH 3, to which 0.100 mL of 100 micrograms/mL chloramine-T had been added. The acidic buffer was then made basic by the addition of 0.200 mL of 1 M NaOH followed by 0.100 mL of isoamyl alcohol. To this solution, 5.0 mL of pentane was added and the biphasic mixture was shaken for 30 minutes followed by centrifugation at about 2000 x g. The pentane was pipetted into a small vial, reduced to dryness with a stream of nitrogen and reconstituted in HPLC mobile phase. An aliquot was then analyzed by HPLC utilizing a spectrophotometric ultraviolet detector. Compounds of interest in this case were quantified by observation at 254 nm.

HPLC analysis may be accomplished with many different mobile phases. The example tracings presented in Figure 1 utilized a 3 µm silica stationary phase in a column of 6 mm x 15 cm. The mobile phase consisted of a mixture of 80 parts of 25 mM NaH₂PO₄, pH 3.0, 20 parts of acetonitrile, and was doped with 21 mM n-nonylamine. The column exhibited approximately 150,000 theoretical plates per meter in a test system used by the manufacturer. Figure 1 illustrates that desmethylchlorpromazine, a metabolite of chlorpromazine, elutes in the area of the chromatogram in which the analytes and internal standard elute. Treatment with chloramine-T completely eliminated the desmethylchlorpromazine as an interfering substance.

### EXAMPLE 3

### Fluorescent Polarization Immunoassay For Total Doxepins In The Presence Of Phenothiazines

The present example illustrates that chlorpromazine (CPZ) is significantly recognized in the immunoassay for doxepin and desdoxepin (Total DOX) and gives incorrect readings for the quantitation of Total DOX (Table II). These data also show that including chloramine-T in the procedure removes the recognition of CPZ in the immunoassay for Total DOX.

To a 0.25 mL solution containing Total DOX and CPZ was added 0.90 mL of heptane:isoamyl alcohol (35:1, v/v) solution and 0.100 mL of sodium carbonate solution. After vortexing for 60 seconds and centrifuging for 30 seconds, 0.50 mL of the upper phase (heptane:isoamyl alcohol phase) was transferred to a clean tube. Next, 0.100 mL of HCl (0.05 N) and 0.040 mL of either distilled water or chloramine-T (0.040 mg/mL) in distilled water were added. After vortexing for 30 seconds, waiting 120 seconds, and centrifuging for 30 seconds, 0.085 mL of the lower phase (aqueous phase of HCl and water with or without chloramine-T) was transferred for quantitation by immunoassay for Total DOX employing an Abbott TDx analytical system.

In a sample containing Total DOX at 100 ng/mL and CPZ at 1000 ng/mL, the concentration in the immunoassay without the addition of chloramine-T was 232 ng/mL or an error of more than twofold as shown in Table II below. The addition of chloramine-T removed the recognition of CPZ by the immunoassay for Total DOX and a value of 95 ng/mL was obtained. This procedure allows quantitation of Total DOX by immunoassay in samples containing CPZ.

**TABLE II**

| Removal of Chlorpromazine Interferant In Abbott TDx Total DOX FPIA | | | |
|---|---|---|---|
| Amount Total DOX Added | Amount CPZ Added | Concentration of Total DOX when the procedure includes: | |
| | | Water | Chloramine-T |
| 0 | 1000 | 143 | 7 |
| 0 | 2000 | 197 | 10 |
| 50 | 1000 | 181 | 49 |
| 75 | 1000 | 199 | 83 |
| 100 | 1000 | 232 | 95 |
| 100 | 0 | 104 | 101 |
| 125 | 500 | 223 | 135 |
| 150 | 1000 | 274 | 155 |
| [all concentrations in ng/mL] | | | |

### EXAMPLE 4

### Fluorescent Polarization Immunoassay For Amitriptyline and Nortriptyline

Chlorpromazine (CPZ) crossreacts with the antibodies used in the FPIA determinations of amitriptyline (AMI) and nortriptyline (NOR), which lead to significant overestimations of AMI and NOR levels. Treatment of a test sample with chloramine-T according to the present invention modifies oxidatively the structure of chlorpromazine in such a fashion that it no longer crossreacts in either immunoassay.

A test sample (0.200 mL) was made basic by the addition of 0.050 mL of 1 N NaOH and dissociated from serum proteins by addition of 0.050 mL isoamyl alcohol; this mixture was briefly mixed and allowed to stand for at least 5.0 minutes, after which time 0.500 mL of n-heptane was added and the sample vortexed vigorously for 30 seconds. The sample/n-heptane biphasic mixture was centrifuged at 10,000 x g for 1 minute to clarify the phases. A measured 0.400 mL aliquot of the upper (n-heptane) phase was then transferred to a new tube with 0.400 mL of 0.1 M glycyl-glycine buffer (pH 3) and vortexed vigorously for 30 sec. Forty microliters (0.040 mL) of chloramine-T solution (100 ug/mL) was then added to the biphasic mixture (chloramine-T treatment as shown in Table III); in the control condition (no treatment), 0.040 mL of extraction buffer was added in place of chloramine-T. Samples were centrifuged at 10,000 x g for 1 minute, with the bottom (glycyl-glycine) phase taken for analysis. Treated and untreated samples were run in duplicate on the Abbott TDx analytical system, and concentrations were determined using stored calibration curves for either AMI or NOR.

**TABLE III**

| Removal of Chlorpromazine Interferant In Abbott TDx Amitriptyline and Nortriptyline FPIA | | |
|---|---|---|
| SAMPLE | TREATMENT | CONCENTRATION |
| AMI | NONE | 75 ng/mL |
| AMI + CPZ | NONE | 300 ng/mL |
| AMI + CPZ | Chloramine-T | 78 ng/mL |
| NOR | NONE | 65 ng/mL |
| NOR + CPZ | NONE | 101 ng/mL |
| NOR + CPZ | Chloramine-T | 73 ng/mL |
| [CPZ concentration = 1,000 ng/mL] | | |

Accordingly, treatment with chloramine-T allowed for the correct determination of AMI and NOR levels in the presence of high concentrations of chlorpromazine.

### EXAMPLE 5

### High Pressure Liquid Chromatography Determination of Amitriptyline and Nortriptyline

In the HPLC analysis of tricyclic antidepressants such as amitriptyline and nortriptyline, phenothiazine drugs such as chlorpromazine or its metabolite desmethylchlorpromazine may interfere with analysis of these antidepressant drugs or their hydroxylated metabolites. Individual 0.400 mL aliquots containing nortriptyline, 10-hydroxyamitriptyline, amitriptyline, desmethylchlorpromazine, and chlorpromazine were treated with either 0.040 mL of chloramine-T solution (100 ug/mL) or 0.040 mL of water (Figure 2). In each instance, the total sample (0.440 mL) was made basic by the addition of 0.050 mL of 1N sodium hydroxide, and 1.0 mL of n-pentane was then added. The biphasic mixture was vortexed vigorously for 1 minute and centrifuged at 10,000 x g for 1 minute. The pentane layer was transferred in its entirety to a conical test tube, reduced to dryness with a stream of nitrogen and reconstituted in HPLC mobile phase. An aliquot was then analyzed by HPLC using spectrophotometric ultraviolet detection at 254 nm. The solid- and mobile-phase constituents for this HPLC analytical procedure were as described in Example 2 above.

### EXAMPLE 6

### Removal of Chlorpromazine Interferant By Selenium Dioxide In The Presence of Hydrogen Peroxide

A test sample (0.100 mL) containing imipramine was rendered basic by the addition of 0.100 mL of 0.25 M NaOH and dissociated from serum proteins by the addition of 0.025 mL of isoamyl alcohol. The mixture was allowed to stand for up to 5.0 minutes, after which time 0.50 mL of nonane was added and the sample vortexed vigorously for 1.0 minute. The mixture was then centrifuged for up to 5.0 minutes at approximately 2000 x g to clarify the phases. A 0.100 mL aliquot of the nonane upper phase was then transferred to a tube containing 0.100 mL of an acidic buffer containing 100 ug/mL of selenium dioxide (SeO₂). To this biphasic mixture, 20 uL of a 30% aqueous solution of H₂O₂ was added and the mixture was vortexed, ninety microliters (0.090 mL) of the lower aqueous phase was then transferred to the sample wells and analyzed by the Abbott TDx analytical system (Table IV). Treatment of the sample extract with selenium dioxide and hydrogen peroxide modified the chemical structure of chlorpromazine such that it was no longer an assay interferant whereas selenium dioxide in the absence of hydrogen peroxide did not cause the conversion of chlorpromazine to chlorpromazine sulfoxide or sulfone.

**TABLE IV**

| Removal Of Chlorpromazine Interferant In FPIA For Imipramine | | |
|---|---|---|
| Sample | Treatment | Mass Measured |
| IMI | NONE | 15 ng/mL |
| IMI+312 ng CPZ | NONE | 98 ng/mL |
| IMI+312 ng CPZ | SeO₂ + H₂O₂ | 14.7 ng/mL |

## Claims (Claims for the following Contracting State(s): AT, BE, CH, LI, DE, DK, FR, GB, GR, IT, LU, MC, NL, SE)

1. An immunoassay method for determining the amount of a tricyclic antidepressant drug in a biological test sample, wherein said tricyclic antidepressant drug does not contain a sulfur moiety and wherein said biological test sample includes a sulfur-containing compound having a chemical structure which is similar to said tricyclic antidepressant drug and is immunologically crossreactive with an antibody to said antidepressant drug, said method comprising the steps of:
(a) forming a pretreatment solution by contacting said biological test sample with an oxidative reagent capable of modifying the chemical structure of said sulfur-containing compound by selectively oxidizing the sulfur moiety of said sulfur-containing compound without modifying the chemical structure of said tricyclic antidepressant drug, whereby crossreactive binding of said antibody to said tricyclic antidepressant drug to said modified sulfur-containing compound is reduced;
(b) contacting said pretreatment solution with (i) a labeled reagent comprising said tricyclic antidepressant drug or derivative thereof labeled with a fluorescent moiety, and (ii) said antibody capable of binding to said labeled reagent and said tricyclic antidepressant drug from said biological test sample to form a reaction solution therewith, said labeled reagent capable of producing a detectable fluorescence polarization response to the presence of said antibody; and
(c) detecting the fluorescence polarization response to said reaction solution as a function of the amount of said tricyclic antidepressant drug present in said test sample.

2. A high pressure liquid chromatography (HPLC) method for determining the amount of a tricyclic antidepressant drug in a biological test sample, wherein said tricyclic antidepressant drug does not contain a sulfur moiety and wherein said biological test sample includes a sulfur-containing compound having a chemical structure similar to the chemical structure of said tricyclic antidepressant drug and an elution profile which interferes with the elution profile of said tricyclic antidepressant drug, said method comprising the steps of:
(a) forming an admixture of an aliquot of said biological test sample and an internal standard;
(b) forming a pretreatment solution by contacting said admixture with an oxidative reagent capable of modifying the chemical structure of said sulfur-containing compound by selectively oxidizing the sulfur moiety of said sulfur-containing compound without modifying the chemical structure of said tricyclic antidepressant drug, whereby the elution profile of said modified sulfur-containing compound does not interfere with the elution profile of said tricyclic antidepressant drug;
(c) contacting said pretreatment solution to an HPLC stationary phase and eluting therefrom with a mobile phase; and
(d) measuring elution times and absorbances of said internal standard and said tricyclic antidepressant drug as they elute from the stationary phase in order to determine the amount of said tricyclic antidepressant drug in said biological test sample.

3. The method of Claim 1 or 2 wherein said biological test sample is selected from the group consisting of whole blood, urine, serum, plasma, cerebrospinal fluid and saliva.

4. The method of Claim 1 or 2 wherein said oxidative reagent is hydrogen peroxide which is generated *in situ*.

5. The method of Claim 1 or 2 wherein said oxidative reagent is selected from the group consisting of N-chlorosulfonamides, hypochlorites, hypobromites, peroxides, peracids, and oxidative enzymes.

6. The method of Claim 1 or 2 wherein said oxidative reagent is a sodium salt of N-chloro-4-methylbenzene sulfonamide.

7. The method of Claim 1 or 2 wherein said tricyclic antidepressant drug is selected from the group consisting of amitriptylene, nortriptylene, imipramine, desipramine, protriptyline, doxepin, desdoxepin, and clomipramine.

8. The method of Claim 1 or 2 wherein said sulfur-containing substance is a phenothiazine selected from the group consisting of chloropromazine, desmethylchloropromazine, thioridazine, desmethylthioridazine, sulforidazine, and mesoridazine.

9. A test kit useful for the fluorescent polarization immunoassay determination of a tricyclic antidepressant drug present in a test sample, wherein said antidepressant drug does not contain a sulfur moiety and wherein said test sample includes a sulfur-containing compound having a chemical structure which is similar to said tricyclic antidepressant drug and is immunologically crossreactive with an antibody to said antidepressant drug, said test kit comprising (a) an oxidative reagent, or reagents which can generate hydrogen peroxide as an oxidative reagent in the test sample, said oxidative reagent being capable of modifying the chemical structure of said sulfur-containing compound by selectively oxidizing the sulfur moiety of said sulfur-containing compound without modifying the chemical structure of said tricyclic antidepressant drug, whereby crossreactive binding of said antibody to said tricyclic antidepressant drug to said modified sulfur-containing compound is reduced and (b) all essential additional reagents for performing the fluorescent polarization immunoassay determination including an antibody to said tricyclic antidepressant drug and a suitable fluorescent tracer compound.

10. A test kit useful for the chromatographic determination of a tricyclic antidepressant drug present in a test sample, wherein said antidepressant drug does not contain a sulfur moiety and wherein said test sample includes a sulfur-containing compound having a chemical structure which is similar to said tricyclic antidepressant drug and an elution profile which interferes with the elution profile of said tricyclic antidepressant drug, said test kit comprising (a) an oxidative reagent, or reagents which can generate hydrogen peroxide as an oxidative reagent in the test sample, said oxidative reagent being capable of modifying the chemical structure of said sulfur-containing compound by selectively oxidizing the sulfur moiety of said sulfur-containing compound without modifying the chemical structure of said tricyclic antidepressant drug, whereby the elution profile of said modified sulfur-containing compound does not interfere with the elution profile of said tricyclic antidepressant drug; and (b) all essential additional reagents for performing the chromatographic determination including an internal standard.

11. The test kit of Claim 10 useful in a high pressure liquid chromatographic determination of said drug; or useful in a thin layer chromatographic determination of said drug.

12. The test kit of any one of Claims 9-11 wherein said oxidative reagent is selected from the group consisting of N-chlorosulfonamides, hypochlorites, hypobromites, peroxides, peracids and oxidative enzymes.

13. The test kit of any one of Claims 9-11 wherein said oxidative reagent is selected from the group consisting of sodium hypochlorite, catalase, perselenic acid and a sodium salt of N-chloro-4-methylbenzene sulfonamide.

14. The test kit of any one of Claims 9-11 wherein said oxidative reagent is a sodium salt of N-chloro-4-methylbenzene sulfonamide or a sodium salt of N-chlorobenzene sulfonamide.

15. The test kit of any one of Claims 9-11 wherein said sulfur-containing substances are phenothiazines.

## Claims (Claims for the following Contracting State(s): ES)

1. An immunoassay method for determining the amount of a tricyclic antidepressant drug in a biological test sample, wherein said tricyclic antidepressant drug does not contain a sulfur moiety and wherein said biological test sample includes a sulfur-containing compound having a chemical structure which is similar to said tricyclic antidepressant drug and is immunologically crossreactive with an antibody to said antidepressant drug, said method comprising the steps of:
(a) forming a pretreatment solution by contacting said biological test sample with an oxidative reagent capable of modifying the chemical structure of said sulfur-containing compound by selectively oxidizing the sulfur moiety of said sulfur-containing compound without modifying the chemical structure of said tricyclic antidepressant drug, whereby crossreactive binding of said antibody to said tricyclic antidepressant drug to said modified sulfur-containing compound is reduced;
(b) contacting said pretreatment solution with (i) a labeled reagent comprising said tricyclic antidepressant drug or derivative thereof labeled with a fluorescent moiety, and (ii) said antibody capable of binding to said labeled reagent and said tricyclic antidepressant drug from said biological test sample to form a reaction solution therewith, said labeled reagent capable of producing a detectable fluorescence polarization response to the presence of said antibody; and
(c) detecting the fluorescence polarization response to said reaction solution as a function of the amount of said tricyclic antidepressant drug present in said test sample.

2. A high pressure liquid chromatography (HPLC) method for determining the amount of a tricyclic antidepressant drug in a biological test sample, wherein said tricyclic antidepressant drug does not contain a sulfur moiety and wherein said biological test sample includes a sulfur-containing compound having a chemical structure similar to the chemical structure of said tricyclic antidepressant drug and an elution profile which interferes with the elution profile of said tricyclic antidepressant drug, said method comprising the steps of:
(a) forming an admixture of an aliquot of said biological test sample and an internal standard;
(b) forming a pretreatment solution by contacting said admixture with an oxidative reagent capable of modifying the chemical structure of said sulfur-containing compound by selectively oxidizing the sulfur moiety of said sulfur-containing compound without modifying the chemical structure of said tricyclic antidepressant drug, whereby the elution profile of said modified sulfur-containing compound does not interfere with the elution profile of said tricyclic antidepressant drug;
(c) contacting said pretreatment solution to an HPLC stationary phase and eluting therefrom with a mobile phase; and
(d) measuring elution times and absorbances of said internal standard and said tricyclic antidepressant drug as they elute from the stationary phase in order to determine the amount of said tricyclic antidepressant drug in said biological test sample.

3. The method of Claim 1 or 2 wherein said biological test sample is selected from the group consisting of whole blood, urine, serum, plasma, cerebrospinal fluid and saliva.

4. The method of Claim 1 or 2 wherein said oxidative reagent is hydrogen peroxide which is generated *in situ*.

5. The method of Claim 1 or 2 wherein said oxidative reagent is selected from the group consisting of N-chlorosulfonamides, hypochlorites, hypobromites, peroxides, peracids, and oxidative enzymes.

6. The method of Claim 1 or 2 wherein said oxidative reagent is a sodium salt of N-chloro-4-methylbenzene sulfonamide.

7. The method of Claim 1 or 2 wherein said tricyclic antidepressant drug is selected from the group consisting of amitriptylene, nortriptylene, imipramine, desipramine, protriptyline, doxepin, desdoxepin, and clomipramine.

8. The method of Claim 1 or 2 wherein said sulfur-containing substance is a phenothiazine selected from the group consisting of chloropromazine, desmethylchloropromazine, thioridazine, desmethylthioridazine, sulforidazine, and mesoridazine.

9. Use of a test kit for the fluorescent polarization immunoassay determination of a tricyclic antidepressant drug present in a test sample, wherein said antidepressant drug does not contain a sulfur moiety and wherein said test sample includes a sulfur-containing compound having a chemical structure which is similar to said tricyclic antidepressant drug and is immunologically crossreactive with an antibody to said antidepressant drug, said test kit comprising (a) an oxidative reagent, or reagents which can generate hydrogen peroxide as an oxidative reagent in the test sample, said oxidative reagent being capable of modifying the chemical structure of said sulfur-containing compound by selectively oxidizing the sulfur moiety of said sulfur-containing compound without modifying the chemical structure of said tricyclic antidepressant drug, whereby crossreactive binding of said antibody to said tricyclic antidepressant drug to said modified sulfur-containing compound is reduced and (b) all essential additional reagents for performing the fluorescent polarization immunoassay determination including an antibody to said tricyclic antidepressant drug and a suitable fluorescent tracer compound.

10. Use of a test kit for the chromatographic determination of a tricyclic antidepressant drug present in a test sample, wherein said antidepressant drug does not contain a sulfur moiety and wherein said test sample includes a sulfur-containing compound having a chemical structure which is similar to said tricyclic antidepressant drug and an elution profile which interferes with the elution profile of said tricyclic antidepressant drug, said test kit comprising (a) an oxidative reagent, or reagents which can generate hydrogen peroxide as an oxidative reagent in the test sample, said oxidative reagent being capable of modifying the chemical structure of said sulfur-containing compound by selectively oxidizing the sulfur moiety of said sulfur-containing compound without modifying the chemical structure of said tricyclic antidepressant drug, whereby the elution profile of said modified sulfur-containing compound does not interfere with the elution profile of said tricyclic antidepressant drug; and (b) all essential additional reagents for performing the chromatographic determination including an internal standard.

11. The use of Claim 10 in a high pressure liquid chromatographic determination of said drug; or in a thin layer chromatographic determination of said drug.

12. The use of any one of Claims 9-11 wherein said oxidative reagent is selected from the group consisting of N-chlorosulfonamides, hypochlorites, hypobromites, peroxides, peracids and oxidative enzymes.

13. The use of any one of Claims 9-11 wherein said oxidative reagent is selected from the group consisting of sodium hypochlorite, catalase, perselenic acid and a sodium salt of N-chloro-4-methylbenzene sulfonamide.

14. The use of any one of Claims 9-11 wherein said oxidative reagent is a sodium salt of N-chloro-4-methylbenzene sulfonamide or a sodium salt of N-chlorobenzene sulfonamide.

15. The use of any one of Claims 9-11 wherein said sulfur-containing substances are phenothiazines.

## Patentansprüche (Patentansprüche für folgende(n) Vertragsstaat(en): AT, BE, CH, LI, DE, DK, FR, GB, GR, IT, LU, MC, NL, SE)

1. Ein Immunoassayverfahren zur Bestimmung der Menge eines trizyklischen Antidepressivums in einer biologischen Testprobe, worin das trizyklische Antidepressivum keine Schwefelkomponente enthält und worin die biologische Testprobe eine schwefelenthaltende Verbindung enthält, die eine chemische Struktur hat, die dem trizyklischen Antidepressivum ähnlich ist und immunologisch kreuzreaktiv ist mit einem Antikörper zu dem Antidepressivum, wobei das Verfahren folgende Schritte umfaßt:
(a) Bilden einer Vorbehandlungslösung, indem die biologische Testprobe mit einem oxidativen Reagens in Kontakt gebracht wird, das in der Lage ist, die chemische Struktur der schwefelenthaltenden Verbindung zu modifizieren, indem die Schwefelkomponente der schwefelenthaltenden Verbindung selektiv oxidiert wird, ohne die chemische Struktur des trizyklischen Antidepressivums zu modifizieren, wobei die kreuzreaktive Bindung des Antikörpers zu dem Antidepressivum gegenüber der modifizierten schwefelenthaltenden Verbindung reduziert ist;
(b) In-Kontakt-Bringen der Vorbehandlungslösung mit (i) einem markierten Reagenz, das das trizyklische Antidepressivum oder ein Derivat davon, markiert mit einer fluoreszierenden Komponente, enthält und (ii) wobei der Antikörper in der Lage ist, das markierte Reagenz und das trizyklische Antidepressivum aus der biologischen Testprobe zu binden, damit eine Reaktionslösung gebildet wird, wobei das markierte Reagens in der Lage ist, eine detektierbare Fluoreszenzpolarisationsantwort auf die Anwesenheit des Antikörpers zu geben; und
(c) Detektieren der Fluoreszenzpolarisationsantwort auf die Reaktionslösung als eine Funktion der Menge des trizyklischen Antidepressivums, die in der Testprobe vorhanden ist.

2. Ein Hochleistungsflüssigchromatographieverfahren (HPLC) zur Bestimmung der Menge eines trizyklischen Antidepressivums in einer biologischen Testprobe, worin das trizyklische Antidepressivum keine Schwefelkomponente enthält und worin die biologische Testprobe eine schwefelenthaltende Verbindung enthält, die eine chemische Struktur ähnlich der chemischen Struktur des trizyklischen Antidepressivums hat, und ein Elutionsprofil, das das Elutionsprofil des trizyklischen Antidepressivums beeinflußt, wobei das Verfahren folgende Schritte umfaßt:
(a) Bilden einer Mischung aus einem Aliquot der biologischen Testprobe und einem internen Standard;
(b) Bilden einer Vorbehandlungslösung, indem die Mischung mit einem oxidativen Reagens in Kontakt gebracht wird, das in der Lage ist, die chemische Struktur der schwefelenthaltenden Verbindung durch selektive Oxidation der Schwefelkomponente der schwefelenthaltenden Verbindung zu modifizieren, ohne die chemische Struktur des trizyklischen Antidepressivums zu modifizieren, durch das Elutionsprofil der modifizierten schwefelenthaltenden Verbindung das Elutionsprofil des trizyklischen Antidepressivums nicht beeinflußt;
(c) In-Kontakt-Bringen der Vorbehandlungslösung mit einer HPLC stationären Phase und daraus Eluieren mit einer mobilen Phase; und
(d) Messen von Elutionszeiten und Absorptionen von dem internen Standard und dem trizyklischen Antidepressivum während sie von der stationären Phase eluieren, um die Menge des trizyklischen Antidepressivums in der biologischen Testprobe zu bestimmen.

3. Das Verfahren nach Anspruch 1 oder 2, worin die biologische Testprobe gewählt ist aus der Gruppe bestehend aus Vollblut, Urin, Serum, Plasma, Cerebrospinalflüssigkeit und Speichel.

4. Das Verfahren nach Anspruch 1 oder 2, worin das oxidative Reagens Wasserstoffperoxid ist, das *in situ* hergestellt wird.

5. Das Verfahren nach Anspruch 1 oder 2, worin das oxidative Reagens gewählt ist aus der Gruppe bestehend aus N-Chlorsulfonamid, Hypochloriten, Hypobromiten, Peroxiden, Peraciden und oxidativen Enzymen.

6. Das Verfahren nach Anspruch 1 oder 2, worin das oxidative Reagens ein Natriumsalz von N-Chlor-4-methylbenzensulfonamid ist.

7. Das Verfahren nach Anspruch 1 oder 2, worin das trizyklische Antidepressivum gewählt ist aus der Gruppe bestehend aus Amitriptylen, Nortriptylen, Imipramin, Desipramin, Protriptylin, Doxepin, Desdoxepin und Clomipramin.

8. Das Verfahren nach Anspruch 1 oder 2, worin die schwefelenthaltende Substanz ein Phenothiazin ist, gewählt aus der Gruppe bestehend aus Chlorpromazin, Desmethylchlorpromazin, Thioridazin, Desmethylthioridazin, Sulforidazin und Mesoridazin.

9. Ein Testkit, nützlich für die Fluoreszenzpolarisationsimmunoassaybestimmung eines trizyklischen Antidepressivums, das in einer Testprobe enthalten ist, worin das Antidepressivum keine Schwefelkomponente enthält und worin die Testprobe eine schwefelenthaltende Verbindung enthält, die eine chemische Struktur hat, die dem trizyklischen Antidepressivum ähnlich ist und immunologisch kreuzreaktiv mit einem Antikörper zu dem Antidepressivum ist, wobei der Testkit folgendes enthält (a) ein oxidatives Reagens oder Reagenzien, die Wasserstoffperoxid als oxidatives Reagens in der Testprobe erzeugen können, wobei das oxidative Reagens in der Lage ist, die chemische Struktur der schwefelenthaltenden Verbindung durch selektive Oxidation der Schwefelkomponente der schwefelenthaltenden Verbindung zu modifizieren, ohne die chemische Struktur des trizyklischen Antidepressivums zu modifizeren, wobei kreuzreaktives Binden des Antikörpers zu dem trizyklischen Antidepressivum gegenüber der modifizierten schwefelenthaltende Verbindung reduziert ist und wobei (b) alle wesentlichen zusätzlichen Reagenzien zur Durchführung der Fluoreszenzimmunoassaybestimmung einen Antikörper zu dem trizyklischen Antidepressivum und eine passende Fluoreszenztracerverbindung einschließen.

10. Ein Testkit, nützlich zur chromatographischen Bestimmung eines trizyklischen Antidepressivums, das in einer Testprobe vorhanden ist, worin das Antidepressivum keine Schwefelkomponente enthält und worin die Testprobe eine schwefelenthaltende Verbindung enthält, die eine chemische Struktur hat, die dem trizyklischen Antidepressivum ähnlich ist, und ein Elutionsprofil, das das Elutionsprofil des trizyklischen Antidepressivums beinflußt, wobei das Testkit folgendes enthält (a) ein oxidatives Reagens oder Reagenzien, die Wasserstoffperoxid als oxidatives Reagens in der Testprobe erzeugen können, wobei das oxidative Reagenz in der Lage ist, die chemische Struktur der schwefelenthaltenden Verbindung durch selektive Oxidation der Schwefelkomponente der schwefelenthaltenden Verbindung zu modifizieren, ohne die chemische Struktur des trizyklischen Antidepressivums zu modifizieren, wodurch das Elutionsprofil der modifizierten schwefelenthaltenden Verbindung das Elutionsprofil des trizyklischen Antidepressivums nicht beinflußt; und wobei (b) alle wesentlichen zusätzlichen Reagenzien zur Durchführung der chromatographischen Bestimmung, einen internen Standard einschließen.

11. Das Testkit nach Anspruch 10, nützlich in einer hochdruckflüssigchromatographischen Bestimmung des Arzneimittels; oder nützlich in einer dünnschichtchromatographischen Bestimmung des Arzneimittels.

12. Das Testkit nach irgendeinem der Ansprücke 9-11, worin das oxidative Reagens gewählt ist aus der Gruppe bestehend aus N-Chlorsulfonamiden, Hypochloriten, Hypobromiten, Peroxiden, Peraciden und oxidativen Enzymen.

13. Das Testkit nach irgendeinem der Ansprüche 9-11, worin das oxidative Reagens gewählt ist aus der Gruppe bestehend aus Natriumhypochlorit, Katalase, Perselensäure und einem Natriumsalz von N-Chlor-4-methylbenzensulfonamid.

14. Das Testkit nach igendeinem der Ansprüche 9-11, worin das oxidative Reagens ein Natriumsalz von N-Chlor-4-methylbenzensulfonamid ist oder ein Natriumsalz von N-Chlorbenzensulfonamid.

15. Das Testkit nach irgendeinem der Ansprüche 9-11, worin die schwefelenthaltenden Substanzen Phenothiazine sind.

## Patentansprüche (Patentansprüche für folgende(n) Vertragsstaat(en): ES)

1. Ein Immunoassayverfahren zur Bestimmung der Menge eines trizyklischen Antidepressivums in einer biologischen Testprobe, worin das trizyklische Antidepressivum keine Schwefelkomponente enthält und worin die biologische Testprobe eine schwefelenthaltende Verbindung enthält, die eine chemische Struktur hat, die dem trizyklischen Antidepressivum ähnlich ist und immunologisch kreuzreaktiv ist mit einem Antikörper zu dem Antidepressivum, wobei das Verfahren folgende Schritte umfaßt:
(a) Bilden einer Vorbehandlungslösung, indem die biologische Testprobe mit einem oxidativen Reagens in Kontakt gebracht wird, das in der Lage ist, die chemische Struktur der schwefelenthaltenden Verbindung zu modifizieren, indem die Schwefelkomponente der schwefelenthaltenden Verbindung selektiv oxidiert wird, ohne die chemische Struktur des trizyklischen Antidepressivums zu modifizieren, wobei die kreuzreaktive Bindung des Antikörpers zu dem Antidepressivum gegenüber der modifizierten schwefelenthaltenden Verbindung reduziert ist;
(b) In-Kontakt-Bringen der Vorbehandlungslösung mit (i) einem markierten Reagenz, das das trizyklische Antidepressivum oder ein Derivat davon, markiert mit einer fluoreszierenden Komponente, enthält und (ii) wobei der Antikörper in der Lage ist, das markierte Reagenz und das trizyklische Antidepressivum aus der biologischen Testprobe zu binden, damit eine Reaktionslösung gebildet wird, wobei das markierte Reagens in der Lage ist, eine detektierbare Fluoreszenzpolarisationsantwort auf die Anwesenheit des Antikörpers zu geben; und
(c) Detektieren der Fluoreszenzpolarisationsantwort auf die Reaktionslösung als eine Funktion der Menge des trizyklischen Antidepressivums, die in der Testprobe vorhanden ist.

2. Ein Hochleistungsflüssigchromatographieverfahren (HPLC) zur Bestimmung der Menge eines trizyklischen Antidepressivums in einer biologischen Testprobe, worin das trizyklische Antidepressivum keine Schwefelkomponente enthält und worin die biologische Testprobe eine schwefelenthaltende Verbindung enthält, die eine chemische Struktur ähnlich der chemischen Struktur des trizyklischen Antidepressivums hat, und ein Elutionsprofil, das das Elutionsprofil des trizyklischen Antidepressivums beeinflußt, wobei das Verfahren folgende Schritte umfaßt:
(a) Bilden einer Mischung aus einem Aliquot der biologischen Testprobe und einem internen Standard;
(b) Bilden einer Vorbehandlungslösung, indem die Mischung mit einem oxidativen Reagens in Kontakt gebracht wird, das in der Lage ist, die chemische Struktur der schwefelenthaltenden Verbindung durch selektive Oxidation der Schwefelkomponente der schwefelenthaltenden Verbindung zu modifizieren, ohne die chemische Struktur des trizyklischen Antidepressivums zu modifizieren, durch das Elutionsprofil der modifizierten schwefelenthaltenden Verbindung das Elutionsprofil des trizyklischen Antidepressivums nicht beeinflußt;
(c) In-Kontakt-Bringen der Vorbehandlungslösung mit einer HPLC stationären Phase und daraus Eluieren mit einer mobilen Phase; und
(d) Messen von Elutionszeiten und Absorptionen von dem internen Standard und dem trizyklischen Antidepressivum während sie von der stationären Phase eluieren, um die Menge des trizyklischen Antidepressivums in der biologischen Testprobe zu bestimmen.

3. Das Verfahren nach Anspruch 1 oder 2, worin die biologische Testprobe gewählt ist aus der Gruppe bestehend aus Vollblut, Urin, Serum, Plasma, Cerebrospinalflüssigkeit und Speichel.

4. Das Verfahren nach Anspruch 1 oder 2, worin das oxidative Reagens Wasserstoffperoxid ist, das *in situ* hergestellt wird.

5. Das Verfahren nach Anspruch 1 oder 2, worin das oxidative Reagens gewählt ist aus der Gruppe bestehend aus N-Chlorsulfonamid, Hypochloriten, Hypobromiten, Peroxiden, Peraciden und oxidativen Enzymen.

6. Das Verfahren nach Anspruch 1 oder 2, worin das oxidative Reagens ein Natriumsalz von N-Chlor-4-methylbenzensulfonamid ist.

7. Das Verfahren nach Anspruch 1 oder 2, worin das trizyklische Antidepressivum gewählt ist aus der Gruppe bestehend aus Amitriptylen, Nortriptylen, Imipramin, Desipramin, Protriptylin, Doxepin, Desdoxepin und Clomipramin.

8. Das Verfahren nach Anspruch 1 oder 2, worin die schwefelenthaltende Substanz ein Phenothiazin ist, gewählt aus der Gruppe bestehend aus Chlorpromazin, Desmethylchlorpromazin, Thioridazin, Desmethylthioridazin, Sulforidazin und Mesoridazin.

9. Ein Testkit, nützlich für die Fluoreszenzpolarisationsimmunoassaybestimmung eines trizyklischen Antidepressivums, das in einer Testprobe enthalten ist, worin das Antidepressivum keine Schwefelkomponente enthält und worin die Testprobe eine schwefelenthaltende Verbindung enthält, die eine chemische Struktur hat, die dem trizyklischen Antidepressivum ähnlich ist und immunologisch kreuzreaktiv mit einem Antikörper zu dem Antidepressivum ist, wobei der Testkit folgendes enthält (a) ein oxidatives Reagens oder Reagenzien, die Wasserstoffperoxid als oxidatives Reagens in der Testprobe erzeugen können, wobei das oxidative Reagens in der Lage ist, die chemische Struktur der schwefelenthaltenden Verbindung durch selektive Oxidation der Schwefelkomponente der schwefelenthaltenden Verbindung zu modifizieren, ohne die chemische Struktur des trizyklischen Antidepressivums zu modifizeren, wobei kreuzreaktives Binden des Antikörpers zu dem trizyklischen Antidepressivum gegenüber der modifizierten schwefelenthaltende Verbindung reduziert ist und wobei (b) alle wesentlichen zusätzlichen Reagenzien zur Durchführung der Fluoreszenzimmunoassaybestimmung einen Antikörper zu dem trizyklischen Antidepressivum und eine passende Fluoreszenztracerverbindung einschließen.

10. Ein Testkit, nützlich zur chromatographischen Bestimmung eines trizyklischen Antidepressivums, das in einer Testprobe vorhanden ist, worin das Antidepressivum keine Schwefelkomponente enthält und worin die Testprobe eine schwefelenthaltende Verbindung enthält, die eine chemische Struktur hat, die dem trizyklischen Antidepressivum ähnlich ist, und ein Elutionsprofil, das das Elutionsprofil des trizyklischen Antidepressivums beinflußt, wobei das Testkit folgendes enthält (a) ein oxidatives Reagens oder Reagenzien, die Wasserstoffperoxid als oxidatives Reagens in der Testprobe erzeugen können, wobei das oxidative Reagenz in der Lage ist, die chemische Struktur der schwefelenthaltenden Verbindung durch selektive Oxidation der Schwefelkomponente der schwefelenthaltenden Verbindung zu modifizieren, ohne die chemische Struktur des trizyklischen Antidepressivums zu modifizieren, wodurch das Elutionsprofil der modifizierten schwefelenthaltenden Verbindung das Elutionsprofil des trizyklischen Antidepressivums nicht beinflußt; und wobei (b) alle wesentlichen zusätzlichen Reagenzien zur Durchführung der chromatographischen Bestimmung, einen internen Standard einschließen.

11. Das Testkit nach Anspruch 10, nützlich in einer hochdruckflüssigchromatographischen Bestimmung des Arzneimittels; oder nützlich in einer dünnschichtchromatographischen Bestimmung des Arzneimittels.

12. Das Testkit nach irgendeinem der Ansprücke 9-11, worin das oxidative Reagens gewählt ist aus der Gruppe bestehend aus N-Chlorsulfonamiden, Hypochloriten, Hypobromiten, Peroxiden, Peraciden und oxidativen Enzymen.

13. Das Testkit nach irgendeinem der Ansprüche 9-11, worin das oxidative Reagens gewählt ist aus der Gruppe bestehend aus Natriumhypochlorit, Katalase, Perselensäure und einem Natriumsalz von N-Chlor-4-methylbenzensulfonamid.

14. Das Testkit nach igendeinem der Ansprüche 9-11, worin das oxidative Reagens ein Natriumsalz von N-Chlor-4-methylbenzensulfonamid ist oder ein Natriumsalz von N-Chlorbenzensulfonamid.

15. Das Testkit nach irgendeinem der Ansprüche 9-11, worin die schwefelenthaltenden Substanzen Phenothiazine sind.

## Revendications (Revendications pour l'(les) Etat(s) contractant(s) suivant(s): AT, BE, CH, LI, DE, DK, FR, GB, GR, IT, LU, MC, NL, SE)

1. Méthode de dosage immunologique pour la détermination de la quantité d'un médicament antidépresseur tricyclique dans un échantillon biologique à tester, dans laquelle ledit médicament antidépresseur tricyclique ne contient pas de fragment soufre et dans laquelle ledit échantillon biologique à tester contient un composé contenant du soufre ayant une structure chimique similaire audit médicament antidépresseur tricyclique et présentant une réaction immunologique croisée avec un anticorps dirigé contre ledit médicament antidépresseur, ladite méthode comprenant les étapes de :
(a) la formation d'une solution de prétraitement par la mise en contact dudit échantillon biologique à tester avec un réactif oxydant capable de modifier la structure chimique dudit composé contenant du soufre par oxydation sélective du fragment soufre dudit composé contenant du soufre sans modifier la structure chimique dudit médicament antidépresseur tricyclique, ce qui réduit la fixation par réaction croisée dudit anticorps dirigé contre ledit médicament antidépresseur tricyclique audit composé contenant du soufre modifié ;
(b) la mise en contact de ladite solution de prétraitement avec (i) un réactif marqué contenant ledit médicament antidépresseur tricyclique ou un de ses dérivés marqué avec un fragment fluorescent et (ii) ledit anticorps capable de se fixer audit réactif marqué et audit médicament antidépresseur tricyclique dudit échantillon biologique à tester pour former avec eux une solution réactionnelle, ledit réactif marqué étant capable de produire une réponse de polarisation de fluorescence détectable en présence dudit anticorps ; et
(c) la détection de la réponse de polarisation de fluorescence de ladite solution réactionnelle en fonction de la quantité dudit médicament antidépresseur tricyclique présent dans ledit échantillon à tester.

2. Méthode de chromatographie liquide haute pression (HPLC) pour déterminer la quantité d'un médicament antidépresseur tricyclique dans un échantillon biologique à tester, dans laquelle ledit médicament antidépresseur tricyclique ne contient pas de fragment soufre et dans laquelle ledit échantillon biologique à tester contient un composé contenant du soufre ayant une structure chimique similaire à la structure chimique dudit médicament antidépresseur tricyclique et présentant un profil d'élution interférant avec le profil d'élution dudit médicament antidépresseur tricyclique, ladite méthode comprenant les étapes de :
(a) la formation d'un mélange d'une partie aliquote dudit échantillon biologique à tester et d'un étalon interne ;
(b) la formation d'une solution de prétraitement par la mise en contact dudit mélange avec un réactif oxydant capable de modifier la structure chimique dudit composé contenant du soufre par oxydation sélective du fragment soufre dudit composé contenant du soufre sans modifier la structure chimique dudit médicament antidépresseur tricyclique, le profil d'élution dudit composé contenant du soufre modifié n'interférant ainsi pas avec le profil d'élution dudit médicament antidépresseur tricyclique ;
(c) la mise en contact de ladite solution de prétraitement avec une phase stationnaire de HPLC et son élution avec une phase mobile ; et
(d) la mesure des durées d'élution et des absorbances dudit étalon interne et dudit médicament antidépresseur tricyclique quand ils éluent de la phase stationnaire pour déterminer la quantité dudit médicament antidépresseur tricyclique dans ledit échantillon biologique à tester.

3. Méthode selon la revendication 1 ou 2, dans laquelle ledit échantillon biologique à tester est choisi dans le groupe constitué par le sang total, l'urine, le sérum, le plasma, le fluide cérébro-spinal et la salive.

4. Méthode selon la revendication 1 ou 2, dans laquelle ledit réactif oxydant est du peroxyde d'hydrogène généré *in situ*.

5. Méthode selon la revendication 1 ou 2, dans laquelle ledit réactif oxydant est choisi dans le groupe constitué par les N-chlorosulfonamides, les hypochlorites, les hypobromites, les peroxydes, les peracides et les enzymes oxydantes.

6. Méthode selon la revendication 1 ou 2, dans laquelle ledit réactif oxydant est le sel de sodium du N-chloro-4-méthylbenzènesulfonamide.

7. Méthode selon la revendication 1 ou 2, dans laquelle ledit médicament antidépresseur tricyclique est choisi dans le groupe constitué par l'amitripyline, le nortriptyline, l'imipramine, la désipramine, la protriptyline, la doxépine, la desdoxépine et la clomipramine.

8. Méthode selon la revendication 1 ou 2, dans laquelle la substance contenant du soufre est une phénothiazine choisie dans le groupe constitué par la chloropromazine, la desméthylchloropromazine, la thioridazine, la desméthylthioridazine, la sulforidazine et la mésoridazine.

9. Kit de test utile pour la détermination par dosage immunologique par polarisation de fluorescence d'un médicament antidépresseur tricyclique présent dans un échantillon à tester, ledit médicament antidépresseur ne contenant pas de fragment soufre et ledit échantillon à tester contenant un composé contenant du soufre ayant une structure chimique similaire audit médicament antidépresseur tricyclique et présentant une réaction immunologique croisée avec un anticorps dirigé contre ledit médicament antidépresseur, ledit kit de test comprenant (a) un réactif oxydant, ou des réactifs capables de générer du peroxyde d'hydrogène en tant que réactif oxydant dans l'échantillon à tester, ledit réactif oxydant étant capable de modifier la structure chimique dudit composé contenant du soufre par oxydation sélective du fragment soufre dudit composé contenant du soufre sans modifier la structure chimique dudit médicament antidépresseur tricyclique, ce qui réduit la fixation par réaction croisée dudit anticorps dirigé contre ledit médicament antidépresseur tricyclique audit composé contenant du soufre modifié et (b) tous les autres réactifs essentiels pour la réalisation d'un dosage immunologique par polarisation de fluorescence incluant un anticorps dirigé contre ledit médicament antidépresseur tricyclique et un composé traceur fluorescent approprié.

10. Kit de test utile pour le dosage chromatographique d'un médicament antidépresseur tricyclique présent dans un échantillon à tester, ledit médicament antidépresseur ne contenant pas de fragment soufre et ledit échantillon à tester contenant un composé contenant du soufre ayant une structure chimique similaire audit médicament antidépresseur tricyclique et un profil d'élution interférant avec le profil d'élution dudit médicament antidépresseur tricyclique, ledit kit de test comprenant (a) un réactif oxydant, ou des réactifs capables de générer du peroxyde d'hydrogène en tant que réactif oxydant dans l'échantillon à tester, ledit réactif oxydant étant capable de modifier la structure chimique dudit composé contenant du soufre par oxydation sélective du fragment soufre dudit composé contenant du soufre sans modifier la structure chimique dudit médicament antidépresseur tricyclique, le profil d'élution dudit composé contenant du soufre modifié n'interférant ainsi pas avec le profil d'élution dudit médicament antidépresseur tricyclique ; et (b) tous les autres réactifs essentiels pour la réalisation d'un dosage chromatographique incluant un étalon interne.

11. Kit de test selon la revendication 10 utile dans le dosage dudit médicament par chromatographie liquide à haute pression ; ou utile dans le dosage dudit médicament par chromatographie sur couche mince.

12. Kit de test selon une quelconque des revendications 9 - 11, dans lequel ledit réactif oxydant est choisi dans le groupe constitué par les N-chlorosulfonamides, les hypochlorites, les hypobromites, les peroxydes, les peracides et les enzymes oxydantes.

13. Kit de test selon une quelconque des revendications 9 - 11, dans lequel ledit réactif oxydant est choisi dans le groupe constitué par l'hypochlorite de sodium, une catalase, l'acide persélénique et le sel de sodium du N-chloro-4-méthylbenzènesulfonamide.

14. Kit de test selon une quelconque des revendications 9 - 11, dans lequel ledit réactif oxydant est le sel de sodium du N-chloro-4-méthylbenzènesulfonamide ou le sel de sodium du N-chlorobenzènesulfonamide.

15. Kit de test selon une quelconque des revendications 9 - 11, dans lequel les substances contenant du soufre sont des phénothiazines.

## Revendications (Revendications pour l'(les) Etat(s) contractant(s) suivant(s): ES)

1. Méthode de dosage immunologique pour la détermination de la quantité d'un médicament antidépresseur tricyclique dans un échantillon biologique à tester, dans laquelle ledit médicament antidépresseur tricyclique ne contient pas de fragment soufre et dans laquelle ledit échantillon biologique à tester contient un composé contenant du soufre ayant une structure chimique similaire audit médicament antidépresseur tricyclique et présentant une réaction immunologique croisée avec un anticorps dirigé contre ledit médicament antidépresseur, ladite méthode comprenant les étapes de :
(a) la formation d'une solution de prétraitement par la mise en contact dudit échantillon biologique à tester avec un réactif oxydant capable de modifier la structure chimique dudit composé contenant du soufre par oxydation sélective du fragment soufre dudit composé contenant du soufre sans modifier la structure chimique dudit médicament antidépresseur tricyclique, ce qui réduit la fixation par réaction croisée dudit anticorps dirigé contre ledit médicament antidépresseur tricyclique audit composé contenant du soufre modifié ;
(b) la mise en contact de ladite solution de prétraitement avec (i) un réactif marqué contenant ledit médicament antidépresseur tricyclique ou un de ses dérivés marqué avec un fragment fluorescent et (ii) ledit anticorps capable de se fixer audit réactif marqué et audit médicament antidépresseur tricyclique dudit échantillon biologique à tester pour former avec eux une solution réactionnelle, ledit réactif marqué étant capable de produire une réponse de polarisation de fluorescence détectable en présence dudit anticorps ; et
(c) la détection de la réponse de polarisation de fluorescence de ladite solution réactionnelle en fonction de la quantité dudit médicament antidépresseur tricyclique présent dans ledit échantillon à tester.

2. Méthode de chromatographie liquide haute pression (HPLC) pour déterminer la quantité d'un médicament antidépresseur tricyclique dans un échantillon biologique à tester, dans laquelle ledit médicament antidépresseur tricyclique ne contient pas de fragment soufre et dans laquelle ledit échantillon biologique à tester contient un composé contenant du soufre ayant une structure chimique similaire à la structure chimique dudit médicament antidépresseur tricyclique et présentant un profil d'élution interférant avec le profil d'élution dudit médicament antidépresseur tricyclique, ladite méthode comprenant les étapes de :
(a) la formation d'un mélange d'une partie aliquote dudit échantillon biologique à tester et d'un étalon interne ;
(b) la formation d'une solution de prétraitement par la mise en contact dudit mélange avec un réactif oxydant capable de modifier la structure chimique dudit composé contenant du soufre par oxydation sélective du fragment soufre dudit composé contenant du soufre sans modifier la structure chimique dudit médicament antidépresseur tricyclique, le profil d'élution dudit composé contenant du soufre modifié n'interférant ainsi pas avec le profil d'élution dudit médicament antidépresseur tricyclique ;
(c) la mise en contact de ladite solution de prétraitement avec une phase stationnaire de HPLC et son élution avec une phase mobile ; et
(d) la mesure des durées d'élution et des absorbances dudit étalon interne et dudit médicament antidépresseur tricyclique quand ils éluent de la phase stationnaire pour déterminer la quantité dudit médicament antidépresseur tricyclique dans ledit échantillon biologique à tester.

3. Méthode selon la revendication 1 ou 2, dans laquelle ledit échantillon biologique à tester est choisi dans le groupe constitué par le sang total, l'urine, le sérum, le plasma, le fluide cérébro-spinal et la salive.

4. Méthode selon la revendication 1 ou 2, dans laquelle ledit réactif oxydant est du peroxyde d'hydrogène généré *in situ*.

5. Méthode selon la revendication 1 ou 2, dans laquelle ledit réactif oxydant est choisi dans le groupe constitué par les N-chlorosulfonamides, les hypochlorites, les hypobromites, les peroxydes, les peracides et les enzymes oxydantes.

6. Méthode selon la revendication 1 ou 2, dans laquelle ledit réactif oxydant est le sel de sodium du N-chloro-4-méthylbenzènesulfonamide.

7. Méthode selon la revendication 1 ou 2, dans laquelle ledit médicament antidépresseur tricyclique est choisi dans le groupe constitué par l'amitripyline, le nortriptyline, l'imipramine, la désipramine, la protriptyline, la doxépine, la desdoxépine et la clomipramine.

8. Méthode selon la revendication 1 ou 2, dans laquelle la substance contenant du soufre est une phénothiazine choisie dans le groupe constitué par la chloropromazine, la desméthylchloropromazine, la thioridazine, la desméthylthioridazine, la sulforidazine et la mésoridazine.

9. Utilisation d'un kit de test pour la détermination par dosage immunologique par polarisation de fluorescence d'un médicament antidépresseur tricyclique présent dans un échantillon à tester, ledit médicament antidépresseur ne contenant pas de fragment soufre et ledit échantillon à tester contenant un composé contenant du soufre ayant une structure chimique similaire audit médicament antidépresseur tricyclique et présentant une réaction immunologique croisée avec un anticorps dirigé contre ledit médicament antidépresseur, ledit kit de test comprenant (a) un réactif oxydant, ou des réactifs capables de générer du peroxyde d'hydrogène en tant que réactif oxydant dans l'échantillon à tester, ledit réactif oxydant étant capable de modifier la structure chimique dudit composé contenant du soufre par oxydation sélective du fragment soufre dudit composé contenant du soufre sans modifier la structure chimique dudit médicament antidépresseur tricyclique, ce qui réduit la fixation par réaction croisée dudit anticorps dirigé contre ledit médicament antidépresseur tricyclique audit composé contenant du soufre modifié et (b) tous les autres réactifs essentiels pour la réalisation d'un dosage immunologique par polarisation de fluorescence incluant un anticorps dirigé contre ledit médicament antidépresseur tricyclique et un composé traceur fluorescent approprié.

10. Utilisation d'un kit de test pour la détermination chromatographique d'un médicament antidépresseur tricyclique présent dans un échantillon à tester, ledit médicament antidépresseur ne contenant pas de fragment soufre et ledit échantillon à tester contenant un composé contenant du soufre ayant une structure chimique similaire audit médicament antidépresseur tricyclique et un profil d'élution interférant avec le profil d'élution dudit médicament antidépresseur tricyclique, ledit kit de test comprenant (a) un réactif oxydant, ou des réactifs capables de générer du peroxyde d'hydrogène en tant que réactif oxydant dans l'échantillon à tester, ledit réactif oxydant étant capable de modifier la structure chimique dudit composé contenant du soufre par oxydation sélective du fragment soufre dudit composé contenant du soufre sans modifier la structure chimique dudit médicament antidépresseur tricyclique, le profil d'élution dudit composé contenant du soufre modifié n'interférant ainsi pas avec le profil d'élution dudit médicament antidépresseur tricyclique ; et (b) tous les autres réactifs essentiels pour la réalisation d'un dosage chromatographique incluant un étalon interne.

11. Utilisation selon la revendication 10, dans le dosage dudit médicament par chromatographie liquide à haute pression ; ou dans le dosage dudit médicament par chromatographie sur couche mince.

12. Utilisation selon une quelconque des revendications 9 - 11, dans laquelle ledit réactif oxydant est choisi dans le poupe constitué par les N-chlorosulfonamides, les hypochlorites, les hypobromites, les peroxydes, les peracides et les enzymes oxydantes.

13. Utilisation selon une quelconque des revendications 9 - 11, dans laquelle ledit réactif oxydant est choisi dans le groupe constitué par l'hypochlorite de sodium, une catalase, l'acide persélénique et le sel de sodium du N-chloro-4-méthylbenzènesulfonamide.

14. Utilisation selon une quelconque des revendications 9 - 11, dans laquelle ledit réactif oxydant est le sel de sodium du N-chloro-4-méthylbenzènesulfonamide ou le sel de sodium du N-chlorobenzènesulfonamide.

15. Utilisation selon une quelconque des revendications 9 - 11, dans laquelle les substances contenant du soufre sont des phénothiazines.
